# EUROPEAN PATENT APPLICATION

(11) **EP 4 760 330 A1**
(43) Date of publication of application: **17.06.2026**
(21) Application number: 24219151.8
(22) Date of filing: 11.12.2024
(51) Int. Cl.: G01R 33/56, A61B 5/02, A61B 5/055, A61B 5/00, G06T 7/00

(54) **A SYSTEM AND METHOD UTILISING MAGNETIC RESONANCE DATA TO CHARACTERISE THE MICROSTRUCTURAL COMPOSITION OF BLOOD VESSELS**

(71) Applicant: The Provost, Fellows, Scholars and other Members of Board of Trinity College Dublin, Dublin 2 (IE)
(72) Inventor: The designation of the inventor has not yet been filed
(74) Representative: Purdylucey Intellectual Property

(57) **Abstract**

The present invention relates to computer-implemented methods and systems for characterising the microstructural composition of a blood vessel and the pathological features therein, utilising magnetic resonance quantitative susceptibility mapping and machine learning or Al models. Methods and systems providing explainable quantitative rupture risk assessment of plaques and aneurysm in target blood vessels, particularly carotid arteries, are disclosed. An advantage of the invention is to provide explainable plaque and aneurysm rupture, risk scores based on a single measurement sequence, resulting in a cost, time, and resource efficient risk assessment.

## Description

### Field

The present disclosure relates to a method and system utilising magnetic resonance measurements to determine the microstructural composition of target blood vessels. More particularly, the disclosure relates to the use of machine learning and AI models to identify alterations to the microstructural composition of the target blood vessels and assess the rupture risk of plaque and aneurysms therein, based on magnetic resonance quantitative susceptibility mapping.

### Background

Stroke is the leading cause of disability and the third leading cause of death in the Western World. Most strokes occur in asymptomatic patients and, therefore, new clinical indicators of the disease are required. There are two main types of strokes: ischemic, and haemorrhagic. Ischemic stroke occurs when a blood clot blocks or narrows an artery leading to the brain, reducing blood flow and oxygen to brain cells. Haemorrhagic stroke happens when a blood vessel in the brain bursts, causing bleeding in or around the brain. These latter acute asymptomatic cerebrovascular events are due to factors such as vulnerable plaque or aneurysm rupture. Additionally, aneurysm rupture can cause internal bleeding, which carries significant morbidity and mortality risks.

Whilst significant investment has been made recently in the use of AI in the coordination of care and management of stroke through advanced vascular and neurovascular imaging tools (e.g. RapidAl; VizAl), no significant progress has been made in the timely and accurate detection of increased risk of stroke and/or sudden death from carotid plaque or aneurysm rupture. There is no validated or standardised means of assessing the predisposition of a carotid plaque or an aneurysm to rupture in an individual. Instead, most clinical reports assessing stroke risk rely on the degree of carotid stenosis or aneurysm size, even though this is recognised to be insufficient as an indicator of rupture risk. Despite their increasingly recognised value, specific features of plaques and aneurysms are accounted for in only a minority of cases. Consequently, many people have unnecessary surgery or interventional procedures to remove their plaques or aneurysms whilst others remain undiagnosed and at high risk of stroke.

While the propensity of a plaque or aneurysms to rupture may be influenced by many factors, such as high blood pressure or vessel and atheroma geometry, the specific rupture strength of the artery, fibrous plaque, or aneurysm wall are the most critical aspect in plaque and aneurysms rupture, particularly carotid plaque rupture. Recently, a Carotid Plaque Rupture Risk Reporting and Data System (Plaque RADS), has been proposed as a standardised system for assessing stroke risk in carotid plaque patients. This system requires a complex array of multi-imaging modalities, such as ultrasound, computed tomography, and magnetic resonance imaging, requiring large amounts of time, machinery and qualified staff resources, making this approach non-viable for the large number of potential patients.

There is no available method for determining a plaque or aneurysm rupture risk from a single imaging modality alone. The available techniques are costly and time inefficient, and are contingent on a range of different measurements (i.e. Ultrasound, MRI and CT scans) [Saba et al., Carotid Plaque-RADS: A Novel Stroke Risk Classification System, JACC: Cardiovascular Imaging, Volume 17, Issue 1, 2024, Pages 62-75]. Whilst some Al based carotid plaque studies are emerging based on multi-modal imaging sequences or for identifying symptomatic plaques [Pisu et al., Machine Learning Detects Symptomatic Plaques in Patients With Carotid Atherosclerosis on CT Angiography, Circulation: Cardiovascular Imaging, Volume 17, Issue 6, 2024, Pages e016274], there are currently no available Al based methods for providing an explainable quantitative assessment of plaque or aneurysm rupture risk.

It is an object of the invention to overcome at least one of the above-referenced problems.

### Summary of the Invention

The Applicant has addressed the limitations of the currently available approaches by providing a non-invasive computer implemented technique, which utilises knowledge of microstructural composition in blood vessels and pathologies therein, such as plaques and aneurysms, to determine risk of rupture of these pathologies. Additionally, unique insights into the relationship between plaque or aneurysm characteristics and magnetic resonance information relating to these pathologies is utilised to provide an explainable quantitative rupture risk assessment. The assessment can be utilised in various applications, particularly to reliably determine which plaque and aneurysm patients should undergo surgery. The explainability of the assessment is key for clinical applications, as any 'black box' approaches are known to present considerable barriers in clinical adoption.

The invention uses magnetic resonance (MR) imaging and AI to non-invasively identify a risk of blood vessel plaque or aneurysm rupture. In a preferred embodiment the invention uses quantitative susceptibility mapping (QSM) and AI to non-invasively identify a risk of carotid plaque rupture to identify patients requiring intervention. QSM is particularly sensitive to the load bearing constituents in plaque and aneurysm tissues which are key to reducing the risk of rupture. The invention will be the first QSM based approach utilising AI for determining microstructural composition risks in blood vessel pathologies, including identifying rupture risk in plaques and aneurysms, from key morphological features and, critically, the presence of key loading bearing constituents within the tissue.

In a first aspect, the invention provides a computer implemented method for characterising a microstructural composition of blood vessels, comprising the steps of:
obtaining magnetic resonance information of one or more target blood vessels; and
processing the magnetic resonance information using one or more models, wherein the processing comprises the steps of:
   identifying one or more pathological features in the one or more target bloods vessels; and
   determining feature characteristics of the one or more pathological features.

In an embodiment, the processing the magnetic resonance information using one or more models further comprises the step of calculating a rupture risk of the pathological feature in one or more target blood vessels from the determined feature characteristics of the one or more pathological features.

In an embodiment, the magnetic resonance information comprises magnetic resonance quantitative susceptibility mapping information.

In an embodiment, the one or more pathological features is a plaque.

In an embodiment the method further comprises the step of providing a risk assessment to enable a pathology diagnosis based on the rupture risk of the pathological feature of the one or more target blood vessels.

In an embodiment, the obtaining of the magnetic resonance information comprises non-invasive mapping of the one or more target blood vessels with a magnetic resonance measurement device.

In an embodiment, the one or more target blood vessels is an artery, and the one or more plaques are arterial plaque.

In an embodiment, the artery is a carotid artery, and the arterial plaque is carotid arterial plaque.

In an embodiment, the artery comprises one of: a coronary artery, an aorta, or a cerebral artery.

In an embodiment, the carotid artery plaque is located at the bifurcation of the carotid artery.

In an embodiment, the one or more pathological features is an aneurysm.

In an embodiment, the feature characteristics comprise at least one of:
a presence of a plurality of feature morphologies;
an extent of the plurality of feature morphologies;
a position of the plurality of feature morphologies;
a presence of a plurality of feature constituents;
an extent of the plurality of feature constituents; and
a position of the plurality of feature constituents.

In an embodiment, the plaque characteristics comprise a plurality of plaque morphologies, and wherein the plurality of plaque morphologies comprises one or more of: lipid pools, fibrous caps, calcification, and intraplaque haemorrhages.

In an embodiment, the plaque characteristics comprise a plurality of plaque constituents, and wherein the plurality of plaque constituents comprises a plurality of load-bearing constituents.

In an embodiment, the plurality of load-bearing constituents comprises at least one of: elastin, and collagen.

In an embodiment, the determining feature characteristics of the one or more pathological features utilises measurements of the diamagnetic and paramagnetic susceptibility of at least one of: the one or more feature morphologies, and the one or more feature constituents.

In an embodiment, the magnetic resonance measurement device comprises an MRI scanner.

In an embodiment:
the obtaining of the magnetic resonance information comprises non-invasive mapping of the one or more target blood vessels with a magnetic resonance measurement device; and
the magnetic resonance measurement device is an MRI scanner configured to perform quantitative susceptibility mapping.

In an embodiment, the magnetic resonance information comprises magnetic resonance quantitative susceptibility mapping, and wherein the magnetic resonance measurement device is an MRI scanner configured to perform quantitative susceptibility mapping by acquiring magnitude and phase data from gradient echo sequences with multiple echo times.

In an embodiment, the one or more models utilise cluster analysis to classify the feature characteristics of the one or more pathological features.

In an embodiment, the one or more models comprise a machine learning or AI model.

In an embodiment, the one or more models comprises a deep learning UNET architecture model trained to delineate, one or more of: calcification, intraplaque haemorrhage, collagen rich tissue, collagen poor tissue, elastin rich tissue, and elastin poor tissue in quantitative susceptibility maps.

In an embodiment:
the one or more models are trained using magnetic resonance measurements and physical composition analysis of one or more test blood vessels; and
the one or more test blood vessels are of the same type as the one or more target blood vessels.

In an embodiment, the physical composition analysis comprises one or more of: histological analysis, and biochemical analysis.

In an embodiment, the rupture risk of the pathological feature in the one or more target blood vessels of the one or more target blood vessels comprises a stratified risk score.

In an embodiment:
the magnetic resonance information comprises a plurality of subsets, each corresponding to a cross-sectional slice of the one or more target blood vessels; and
the rupture risk of the pathological feature in the one or more target blood vessels comprises a plurality of sub-risks, each associated with a respective cross-sectional slice of the one or more target blood vessels.

In an embodiment, the method further comprises the step of determining the spatial variation of the rupture risk of the pathological feature in the one or more target blood vessels and the feature characteristics along the length of the one or more target blood vessels based on the plurality of cross-sectional slices of the one or more target blood vessels.

In an embodiment, the method further comprises the step of:
outputting the rupture risk of the pathological feature in the one or more target blood vessels; and
outputting a report comprising the feature characteristics of the one or more pathological features.

In an embodiment:
the magnetic resonance information comprises a plurality of subsets, each corresponding to a cross-sectional slice of the one or more target blood vessels; and
the rupture risk of the pathological feature in the one or more target blood vessels comprises a plurality of sub-risks, each associated with a respective cross-sectional slice of the one or more target blood vessels.

In an embodiment, the method further comprises the step of determining the spatial variation of the rupture risk of the pathological feature in the one or more target blood vessels and the feature characteristics along the length of the one or more target blood vessels based on the plurality of cross-sectional slices of the one or more target blood vessels.

In an embodiment, the invention also provides a system for characterising a microstructural composition of blood vessels, comprising:
a magnetic resonance measurement device configured to determine magnetic resonance information of one or more target blood vessels; and
a computer device, comprising a processor, wherein the processor is configured to execute instructions to:
   obtain magnetic resonance information of one or more target blood vessels utilising the magnetic resonance measurement device; and
   process the magnetic resonance information using one or more models to:
      identify one or more pathological features in the one or more target bloods vessels; and
      determine feature characteristics of the one or more pathological features.

In an embodiment, the processor is configured to execute instructions to further process the magnetic resonance information using one or more models to calculate a risk of rupture of the pathological features of the one or more target blood vessels from the determined feature characteristics of the one or more pathological features.

In an embodiment:
the magnetic resonance measurement device comprises an MRI scanner configured to perform quantitative susceptibility mapping; and
the magnetic resonance information is quantitative susceptibility mapping information.

In an embodiment:
the target blood vessel comprises a carotid artery;
the one or more pathological features comprise a carotid arterial plaque; and
the carotid arterial plaque characteristics comprise a presence, an extent, and a position of one or more of: lipid pools, fibrous caps, calcification, intraplaque haemorrhages, elastin, and collagen.

In an embodiment, the system further comprises a display screen or printer operational connected to the computer device, wherein the processor is further configured to execute instructions to:
output to the display screen or printer the rupture risk of the pathological feature in the one or more target blood vessels; and
output to the display screen or printer a report comprising the feature characteristics of the one or more pathological features.

In an aspect, the invention provides a computer system comprising hardware, software and firm ware for implementing any method detailed above.

In an embodiment of the system, the one or more models comprise a machine learning or AI model trained using magnetic resonance measurements and physical histological analysis of one or more test blood vessels, wherein the one or more test blood vessels are the same type as the one or more target blood vessels.

Other aspects and preferred embodiments of the invention are defined and described in the other claims set out below.

### Brief Description of the Figures

**FIG.1** shows a schematic of an *ex vivo* MR-QSM 120 and histology 130 image registration, as well as the artery and arterial plaque microstructure 140 of a representative carotid sample 111 of a patient 110. This data is used to train a machine learning or AI model 150 modelling the relationship between physical characteristics of plaque determined in histology 130 and data determined from the MR-QSM 120.
**FIG.2** shows an example of image registration and cluster analysis from a representative cadaveric carotid sample. Shown are: Verhoeff's elastin-stained BF (a) and SAF (b) histology slice prior to non-linear image registration; MRI echo-combined magnitude (c), R2* (d), and QSM (e) in the same slice; ROIs determined by k-means clustering (f); Verhoeff's BF (g) and SAF (h) post image registration.
**FIG.3** shows Pearson's correlations between MRI data and microstructural components; from this it can be seen that MR-QSM sequence can identify collagen and elastin fraction in tissue ((a) and (b)) which is not achievable with the standard MR sequences used clinically, i.e. magnitude and R2* imaging (c) - (f).
**FIG.4** shows an *in vivo* QSM neck scan 401 of healthy volunteer 110 illustrating the feasibility of *in vivo* QSM scanning of carotid arteries within an acceptable scan time and resolution (~10 minutes scan time).
**FIG.5** shows an exemplary image postprocessing pipeline 500 for extracting the susceptibility map 507 from an MRI map 501.
**FIG.6** shows the output 600 a machine learning model with a U-Net architecture, illustrating the automatic delineation of plaque regions comprising calcification 601 and intraplaque haemorrhage 602 performed by the model.
**FIG.7** shows an overall workflow diagram 700 of an embodiment of the present invention, including two sub workflows, one for model training 710 based on cadaveric carotid arteries, and one for clinical application 720 utilising *in vivo* MRI maps.

### Detailed Description of the Invention

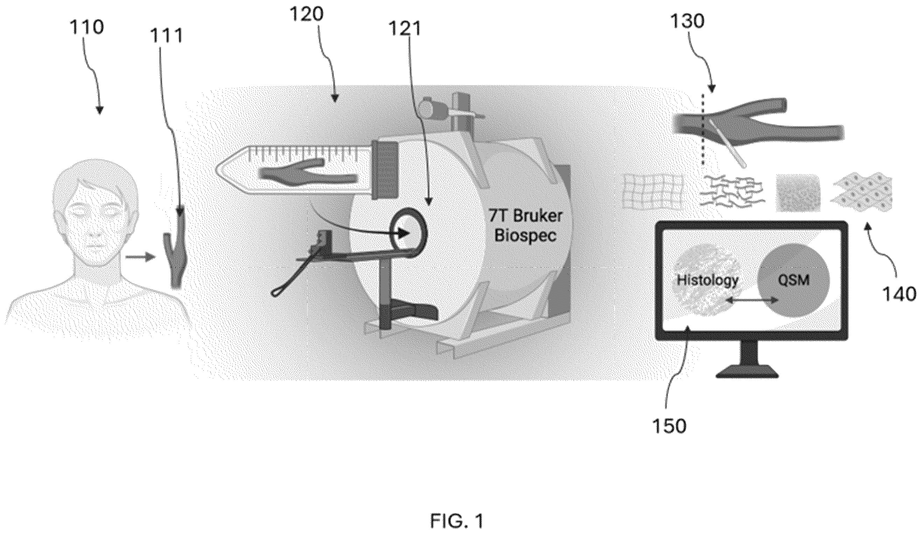

As shown in Figure 1, excised cadaveric carotid arteries 111 (n=5) were scanned using high-resolution MR QSM 121 at 7 Tesla, to illustrate and understand the effect of varying plaque characteristics on the magnetic resonance information obtained by measuring the plaque. After *ex vivo* imaging, image analysis and histology was performed as described in this section below. Similar analysis can be performed for plaques and aneurysms in any other blood vessel, particularly carotid arteries, coronary arteries, aortae, or cerebral arteries.

All carotids were secured, individually, in 50-mL falcon tubes using PLA 3D-printed holders to position them. A total of five scan sessions were performed (one per sample). For QSM, data were acquired using a high-resolution 3D multi-echo gradient-echo (ME-GRE) sequence. Acquisition parameters were: TEs = 5, 13.1, 21.1, 29.3 ms with monopolar readout gradients, TR = 150 ms, flip angle = 30°, bandwidth = 34722 Hz, and 2 averages. The readout direction was oriented along the longitudinal axis of the tube and artery. Using a FOV of 30 mm x 30 mm x 30 mm and a matrix size of 256 x 256 x 256, an isotropic voxel resolution of 0.117 x 0.117 x 0.117 mm3 was achieved. Total scan time for this sequence was 5 hours and 27 minutes.

After MR scanning, the carotids were transferred to histological processing 130. For each carotid, a 2-3 mm long section of the common artery was selected and processed for histological staining. Briefly, all samples were stepwise dehydrated and embedded in paraffin wax. Samples were sectioned into 7-mm thick slices and stained with Haematoxylin & Eosin (H&E, cell content), Verhoeff's elastin (elastin), Picrosirius red (PSR, collagen), and Alizarin red (calcium). A semi-quantitative histological process was carried out to determine the amount of each microstructural component (cells, collagen, elastin, and calcium) in the tissue. An exemplary depiction of the microstructure composition 140 of an artery and arterial plaque is shown, illustrating collagen, elastin, glycosaminoglycans (GAGs) and cells. QuPath software (version 0.2.3) was used to quantify elastin and calcium. Collagen content quantification (collagen fraction) followed a similar pipeline using an in-house developed MATLAB code which uses polarized light microscopy (PLM) to define the collagen areas and PSR-stained BF images to define the tissue area. For cell density analysis, ROIs (see 2.5.2) were manually drawn within QuPath, where the cell detection function was used to yield 2D cell densities.

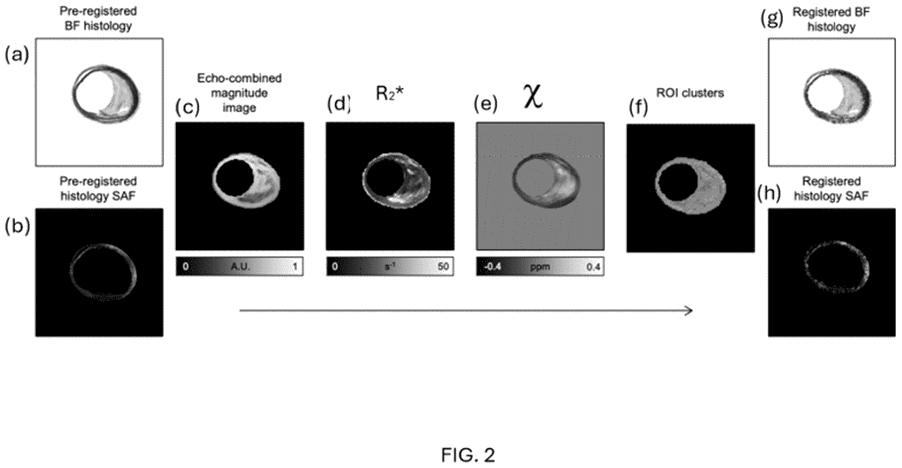

Figure 2 shows an exemplary image registration and cluster analysis from a representative cadaveric carotid sample comprising plaque. Shown are: Verhoeff's elastin-stained BF (Figure 2(a)) and SAF (2(b)) histology slice prior to non-linear image registration; MRI echo-combined magnitude (2(c)), R2* (2(d)), and QSM (X) (2(e)) in the same slice; ROIs determined by k-means clustering (2(f)); Verhoeff's BF (2(g) and SAF (2(h)) post image registration. QSM maps were calculated for each of the carotid arteries. For each sample, coil-combined magnitude images were calculated using the root mean square (RMS) of the channels and coil-combined phase images were produced using the phase difference approach. R2* maps were calculated using the Auto-Regression on Linear Operations algorithm on the coil-combined magnitude data. For masking, an echo-combined magnitude image was calculated using the RMS of all echoes, then thresholded to include all tissue and PBS and exclude the 3D-printed holder and air outside the tube. This mask was manually refined to exclude air bubbles. Field maps were generated using nonlinear field fitting and unwrapped with Laplacian phase unwrapping. The projection-onto-dipole-fields method was used to calculate local field maps using the unwrapped field maps and previously created mask as inputs. Susceptibility maps were calculated using an iterative Tikhonov approach where the regularization parameter (α=0.02) was determined via L-curve optimization. Similar analysis can be performed for plaques and aneurysms contained in any other blood vessel, particularly carotid arteries, coronary arteries, aortae, or cerebral arteries.

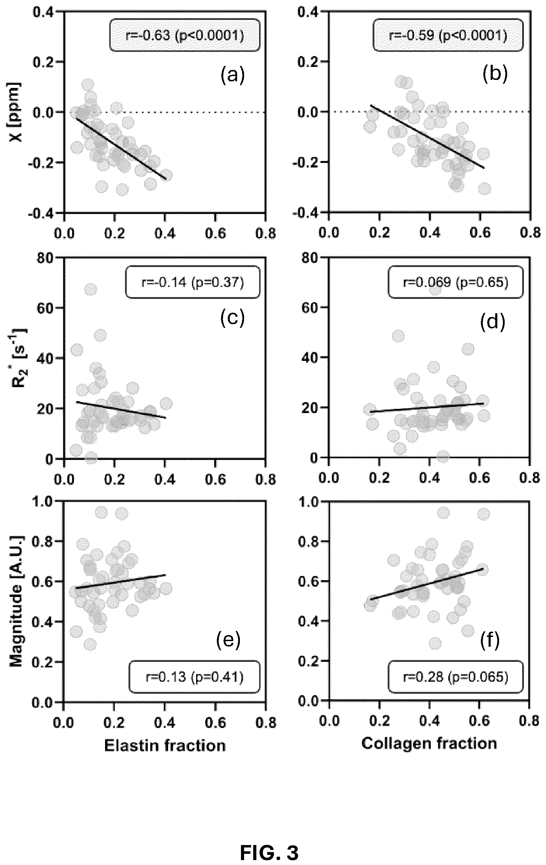

Figure 3 shows the results of the combination of the image registration pipeline described above with semi-quantitative, regional histology analysis to evaluate relationships between MRI and microstructural components of plaque. All samples were brought to histology 130 and stained for elastin, collagen, cells, and calcium. The computed echo-combined magnitude images were used for the correlations and figures.

The Figure 3 graphs illustrate Pearson's correlations between MRI data and microstructural tissue components. Susceptibility values are presented in the top row, R2* in the middle, and magnitude values and their correlation with components are given in the bottom row. The first column is elastin, the second is collagen. Each individual dot corresponds to a singular ROI as defined by k-means clustering (n=9 per sample). Pearson's correlations were performed and presented for the entire dataset. Pearson's coefficients (r) and p values are presented within each plot.

Weak, non-significant (p>0.05) correlations were found between all components and regional magnitude and R2 * measurements (Figures 3(c) - 3(f)). However, a significant, moderate negative correlation between the elastin fraction and regional magnetic susceptibility, rₑₗₐₛₜᵢₙ = -0.63 (p<0.0001) (Figure 3(a)), as well as a significant, moderate negative correlation between collagen and regional magnetic susceptibility, r_{collagen} = -0.59 (p<0.0001) (Figure 3(b)) were found. These results show that the plaque constituents have a relatively large effect on the magnetic resonant information of the blood vessels, in this case carotid arteries. Therefore, the presence, extent and position of the constituents such as elastin and collagen, as well as plaque morphologies, can be determined from magnetic resonance information, particularly QSM.

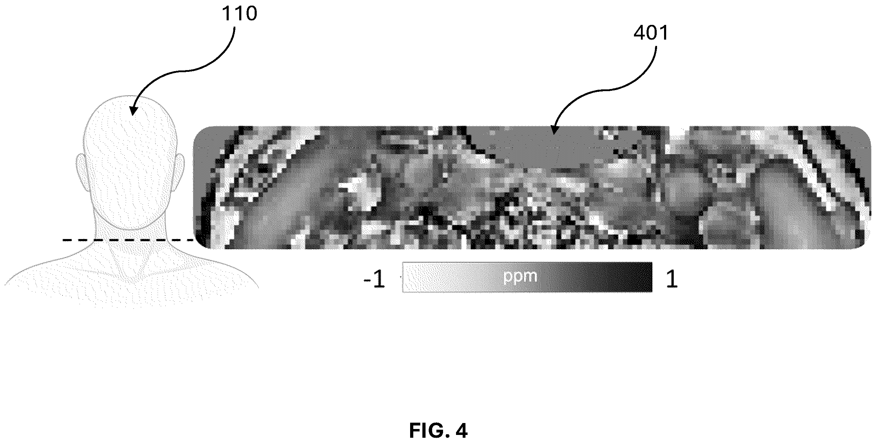

The above-explained approach was used to analyse and determine the relationship between plaque characteristics and QSM measurements. In embodiments according to the current invention, *in vivo* images 401 are used, as shown in Figure 4. Figure 4 illustrates a sample single slice of *in vivo* QSM neck scan 401 of healthy volunteer 110. The example scan 401 establishes the feasibility of *in vivo* QSM scanning of carotid arteries within an acceptable scan time and resolution (~10 minutes scan time). Similar MR QSM measurements can be performed for any other blood vessel that can be scanned to an acceptable resolution in a reasonable scan time, particularly carotid arteries, coronary arteries, aortae, or cerebral arteries. In this way, the analysis of MR QSM measurements characterising the microstructural composition of blood vessels, as well as the plaques and aneurysms therein, can be performed.

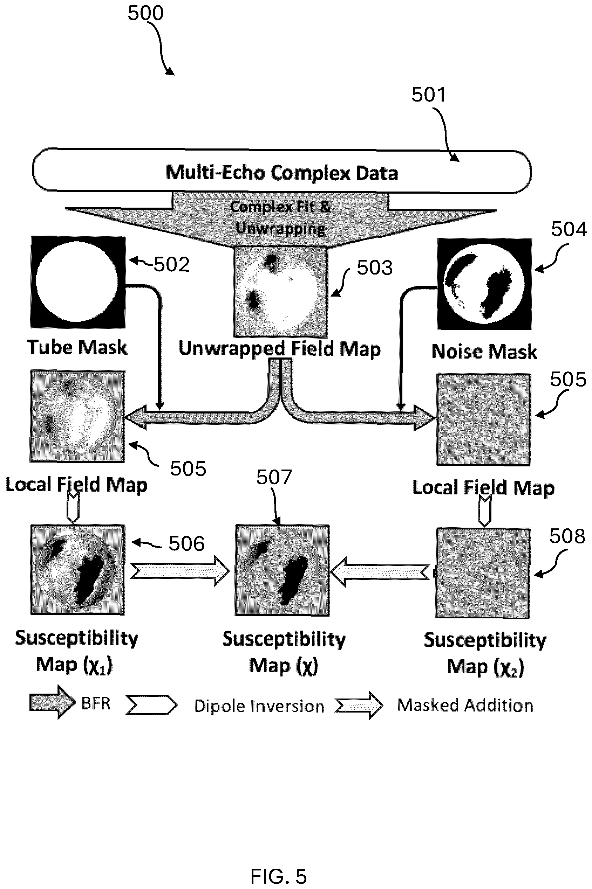

Figure 5 shows an exemplary image postprocessing pipeline 500 for extracting the susceptibility map 507 from the MRI map, such as that shown in Figure 4. Illustrated is a pipeline for Quantitative Susceptibility Mapping (QSM) using multi-echo MRI data 501. It begins with multi-echo complex data 501 acquisition, which undergoes complex fitting and phase unwrapping to generate an unwrapped field map 503. The tube mask 502, used for region selection, and the noise mask 502, which identifies noisy regions, are applied to refine the data and exclude unwanted artifacts.

The local field map 504 is extracted by using the tube mask to focus on the region of interest while excluding the noise. This is followed by background field removal (BFR) to isolate the local magnetic field variation. The local field maps undergo dipole inversion to estimate the susceptibility, leading to initial susceptibility maps 506,507. Finally, masked addition ensures that the susceptibility values are spatially consistent, producing the final susceptibility map 507, highlighting the arterial region. This pipeline can provide a susceptibility map suitable for input into an AI or machine learning model which can be trained to determine the microstructural compositions of any suitable blood vessels and the pathologies therein: in particular plaques and aneurysms in carotid arteries, coronary arteries, aortae, or cerebral arteries.

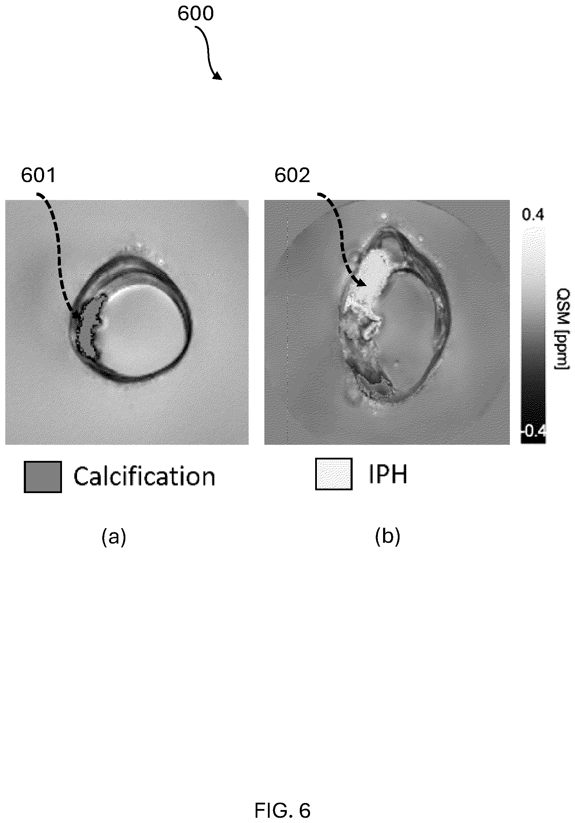

Figure 6 shows two MRI images of carotid arteries, processed and analysed in accordance with an embodiment of the present invention. The images show a QSM MRI map, with the regions segmented by a U-Net convolutional neural network-based model highlighted. The model delineates plaque characteristics, with these images showing regions of calcification 601 and intraplaque haemorrhage (IPH) 602. These images are defined by the QSM, a dimensionless quantity in parts per million, which reflects the relative strength of magnetic susceptibility of various constituents and morphologies. In other embodiments the blood vessel pathology comprises an aneurysm.

In an embodiment of the present invention, a one sequence MR QSM measurement is taken, each slice processed and analysed to provide a plurality of images similar to Figure 6 for analysis. A model trained on the data as described in Figures 1 - 3 is employed to determine the presence, extent and position of plaque or aneurysm characteristics, including constituents and morphologies, such as that shown in Figure 6. The most predictive morphologies for plaques include calcification and intraplaque haemorrhages, and the most predictive constituents include elastin and collagen. Each slice is then provided with a risk score based on the determination of the plaque characteristics in this slice. For example, larger quantities of elastin and collagen indicate a lower risk of plaque or aneurysm rupture. A large plaque or large aneurysm comprising a large amount of calcification may indicate a lower risk of rupture than simpler available approaches would suggest, while a large amount of intraplaque haemorrhage indicates a higher risk of plaque rupture. The rupture risk score for each slice can be combined to produce an overall rupture risk score for the entire plaque or aneurysm region. For example, this may be a weighted average and may weight high risk slices more heavily.

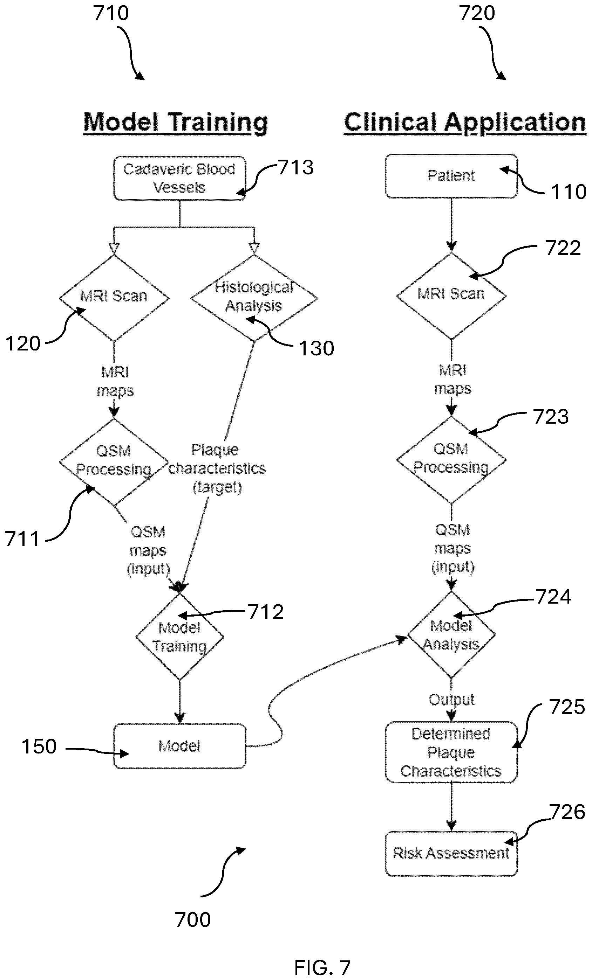

Figure 7 illustrates an overall workflow diagram 700 of the present invention when the blood vessel pathology is plaque, including two sub workflows, one for model training 710, based on cadaveric carotid arteries, and one for clinical application 720.

The model training workflow 710 illustrates an exemplary approach for training a machine learning or AI model 150 to determine target blood vessel pathology characteristics from MRI maps. In this example, the pathologies are plaques. Cadaveric test blood vessels 713 can be obtained and each scanned 120 using *ex vivo* MRI to generate high-resolution magnetic maps of the test blood vessels 713 and any atherosclerotic plaque within it. The MRI data is processed 711 to extract magnetic susceptibility maps of the plaque using Quantitative Susceptibility Mapping (QSM). These susceptibility maps represent the input features for the model training 712, capturing magnetic properties of the plaque that correlate with its composition and pathology. Histological analysis 130 is performed on the same cadaveric test blood vessels 713 to analyse the plaque tissue at a microscopic level and to identify specific plaque characteristics such as lipid content, calcification, IPH, elastin, and collagen. These histological measurements serve as the target variables for model training 712. A predictive model 150 is trained 712 using the magnetic susceptibility maps (input) and the histologically measured plaque characteristics (target). The model 150 is optimized and validated with a subset of the QSM maps and histologically determined plaque characteristics. In a preferred embodiment, the test blood vessels 713 are carotid arteries 111.

The clinical application workflow 720 illustrates an exemplary approach for the clinical use of a system in accordance with an embodiment of the present invention. Data collection is provided from patients 110, by performing *in vivo* MRI scans 722 of live patients to image the target blood vessels 713. In this preferred embodiment the scans 722 are of the neck region and the target blood vessels are carotid arteries. In other embodiments, the workflow could be applied to other blood vessel pathologies. For example, aneurysms in vessels such as coronary arteries, aortae, or cerebral arteries. The relevant data is extracted from the MRI images and processed 723 to generate magnetic susceptibility maps of the plaque using the same QSM technique applied during model training 710. The magnetic susceptibility data from live patients is passed into the previously trained predictive model 150 and analysis 724 is performed. The model 150 outputs estimated plaque characteristics 725 such as lipid content, calcification, IPH, elastin, and collagen. For aneurysms, the model can output characteristics such as wall integrity, thrombus, elastin and collagen content and/or calcification. The predicted plaque characteristics are utilised to determine a risk assessment for the plaque in each patient. This risk score is based on correlations between certain plaque characteristics and clinical risk for conditions like stroke or cardiovascular events. For example, a higher rupture risk score can be correlated with larger quantities of IPH and lower quantities of calcification, elastin, and collagen. For aneurysms, a rupture risk may correlate with thin walls or reduced elastin and collagen content.

The invention provides significant potential for targeting treatment to those at high risk of stroke, as plaque vulnerability is the main risk factor for stroke. The invention can be applied *in vivo* given its non-invasive nature and its efficient scanning time. Only one MR scanning sequence is necessary to obtain all the necessary data, reducing scanning time to less than 30% of that currently required, thus also reducing costs. In addition, the invention removes the need for ionising radiation, as no CT is necessary. An advantage of the methods and systems disclosed herein is to utilise unique insights into the relationship between plaque characteristics and magnetic resonance information to streamline, automate and standardise the process of obtaining a robust reliable and repeatable plaque rupture risk assessment (Rupture Score Index (RSI)), which is quantitative, explainable, repeatable, and accessible.

## Claims

1. A computer implemented method for characterising a microstructural composition of blood vessels, comprising the steps of:
obtaining magnetic resonance information of one or more target blood vessels; and
processing the magnetic resonance information using one or more models, wherein the processing comprises the steps of:
identifying one or more pathological features in the one or more target bloods vessels; and
determining feature characteristics of the one or more pathological features.

2. The computer implemented method of claim 1, wherein the processing the magnetic resonance information using one or more models further comprises the step of calculating a rupture risk of the pathological feature in one or more target blood vessels from the determined feature characteristics of the one or more pathological features

3. The computer implemented method of any previous claim, wherein the magnetic resonance information comprises magnetic resonance quantitative susceptibility mapping information.

4. The computer implemented method of any previous claim, wherein the one or more pathological features is a plaque.

5. The computer implemented method of claim 4, wherein the one or more target blood vessels is a carotid artery, and the plaque is a carotid arterial plaque.

6. The computer implemented method of any of claims 1 to 3, wherein the one or more pathological features is an aneurysm.

7. The computer implemented method of any previous claim, wherein the feature characteristics comprise at least one of:
a presence of a plurality of feature morphologies;
an extent of the plurality of feature morphologies;
a position of the plurality of feature morphologies;
a presence of a plurality of feature constituents;
an extent of the plurality of feature constituents; and
a position of the plurality of feature constituents.

8. The computer implemented method of claim 7, when dependent on claim 4 or claim 5, wherein the plaque characteristics comprise a plurality of plaque morphologies, and wherein the plurality of plaque morphologies comprises one or more of: lipid pools, fibrous caps, calcification, and intraplaque haemorrhages.

9. The computer implemented method of claim 7, when dependent on claim 4 or claim 5, wherein the plaque characteristics comprise a plurality of plaque constituents, and wherein the plurality of plaque constituents comprises a plurality of load-bearing constituents.

10. The computer implemented method of any preceding claim, wherein:
the obtaining of the magnetic resonance information comprises non-invasive mapping of the one or more target blood vessels with a magnetic resonance measurement device; and
the magnetic resonance measurement device is an MRI scanner configured to perform quantitative susceptibility mapping.

11. The computer implemented method of any preceding claim, wherein the one or more models comprises a deep learning UNET architecture model trained to delineate, one or more of: calcification, intraplaque haemorrhage, collagen rich tissue, collagen poor tissue, elastin rich tissue, and elastin poor tissue in quantitative susceptibility maps.

12. The computer implemented method of any of claims 2 to 11, wherein:
the magnetic resonance information comprises a plurality of subsets, each corresponding to a cross-sectional slice of the one or more target blood vessels; and
the rupture risk of the pathological feature in the one or more target blood vessels comprises a plurality of sub-risks, each associated with a respective cross-sectional slice of the one or more target blood vessels.

13. The computer implemented method of any of claims 2 to 12, further comprising the step of:
outputting the rupture risk of the pathological feature in the one or more target blood vessels; and
outputting a report comprising the feature characteristics of the one or more pathological features.

14. A system for characterising a microstructural composition of blood vessels, comprising:
a magnetic resonance measurement device configured to determine magnetic resonance information of one or more target blood vessels; and
a computer device, comprising a processor, wherein the processor is configured to execute instructions to:
obtain magnetic resonance information of one or more target blood vessels utilising the magnetic resonance measurement device; and
process the magnetic resonance information using one or more models to:
identify one or more pathological features in the one or more target bloods vessels; and
determine feature characteristics of the one or more pathological features.

15. A computer device comprising hardware, software and firm ware for implementing the method of claims 1 to 13.
